# EUROPEAN PATENT APPLICATION

(11) **EP 3 498 153 A1**
(43) Date of publication of application: **19.06.2019**
(21) Application number: 17207650.7
(22) Date of filing: 15.12.2017
(51) Int. Cl.: A61B 5/00, A41F 1/00, A44C 5/20, A45C 13/10, F16B 1/00

(54) **AN APPARATUS FOR ATTACHING TO A SUBJECT'S BODY**

(71) Applicant: Nokia Technologies Oy, 02610 Espoo (FI)
(72) Inventor: RADIVOJEVIC, Zoran, Cambridge, Cambridgeshire CB3 0FA (GB)
(74) Representative: Swindell & Pearson Limited

(57) **Abstract**

An apparatus and wearable electronic device wherein the apparatus comprises: a flexible member arranged to be wrapped around part of a subject's body, the flexible member comprising a first end portion and a second end portion; a magnetic arrangement embedded within the flexible member wherein the magnetic arrangement comprises at least one magnet positioned within the first end portion of the flexible member and at least one magnetic portion positioned within the second end portion of the flexible member; and wherein the magnetic arrangement is arranged to hold the first end portion of the flexible member in position relative to the second end portion when the flexible member is bent so that the first end portion and the second end portion overlap.

## Description

### TECHNOLOGICAL FIELD

Examples of the disclosure relate to an apparatus for attaching to a subject's body. In particular they relate to an apparatus that can be adjusted to attach to different subjects or to different parts of the same subject's body.

### BACKGROUND

Apparatus for attaching to a subject's body are known. For example, a cuff or a sleeve could be wrapped around a subject's arm to enable biometric parameters such as blood pressure and heart rate to be monitored. In some examples a strap could be wrapped around a subject's chest or torso to enable the subject's heart rate to be monitored.

### BRIEF SUMMARY

According to various, but not necessarily all, examples of the disclosure there is provided an apparatus comprising: a flexible member arranged to be wrapped around part of a subject's body, the flexible member comprising a first end portion and a second end portion; a magnetic arrangement embedded within the flexible member wherein the magnetic arrangement comprises at least one magnet positioned within the first end portion of the flexible member and at least one magnetic portion positioned within the second end portion of the flexible member; and wherein the magnetic arrangement is arranged to hold the first end portion of the flexible member in position relative to the second end portion when the flexible member is bent so that the first end portion and the second end portion overlap.

The magnetic arrangement may comprise a plurality of magnets arranged in an array comprising a plurality of columns and a plurality of rows.

The magnetic arrangement may comprise a pattern with at least two orders of rotational symmetry.

The magnetic arrangement may comprise a tessellated array comprising a plurality of polygons positioned within the first end portion of the flexible member and a plurality of magnets arranged to align with vertices of the polygons positioned within the second end portion of the flexible member.

The magnetic arrangement may comprise a hexagonal array comprising a plurality of polygons positioned within the first end portion of the flexible member and a plurality of magnets arranged to align with vertices of the hexagons positioned within the second end portion of the flexible member.

A plurality of magnets may be provided within least one portion of the flexible member and the plurality of magnets are arranged so that adjacent magnets within have repelling polarities.

The at least one magnet positioned at the first end portion may have a first polarity and at least one magnet positioned at the second end portion may have a different polarity.

The magnetic arrangement may be printed onto the flexible member.

The at least one magnetic portion positioned within the second end portion of the flexible member may comprise one or more magnets.

The flexible member may be arranged to be wrapped around a portion of a subject's body.

The flexible member may comprise textile.

The flexible member may comprise polymer.

The apparatus may comprise one or more sensors arranged to detect a biometric parameter of the subject.

According to various, but not necessarily all, examples of the disclosure there may be provided wearable electronic device comprising an apparatus as claimed in any preceding claim.

According to various, but not necessarily all, examples of the disclosure, there is provided examples as claimed in the appended claims.

### BRIEF DESCRIPTION

For a better understanding of various examples that are useful for understanding the detailed description, reference will now be made by way of example only to the accompanying drawings in which:
Fig. 1 illustrates an example apparatus;
Figs. 2A and 2B illustrate sections of an example apparatus;
Figs. 3A to 3C illustrate another example apparatus; and
Fig. 4 illustrate an example apparatus in use.

### DETAILED DESCRIPTION

Examples of the disclosure relate to an apparatus 1 for attaching to a subject's body. The apparatus 1 is adjustable so that it can be securely attached to different subjects or to different parts of the same subject's body. The apparatus 1 uses a magnetic arrangement 5 comprising one or more of magnets to enable the apparatus 1 to be adjusted to different sizes.

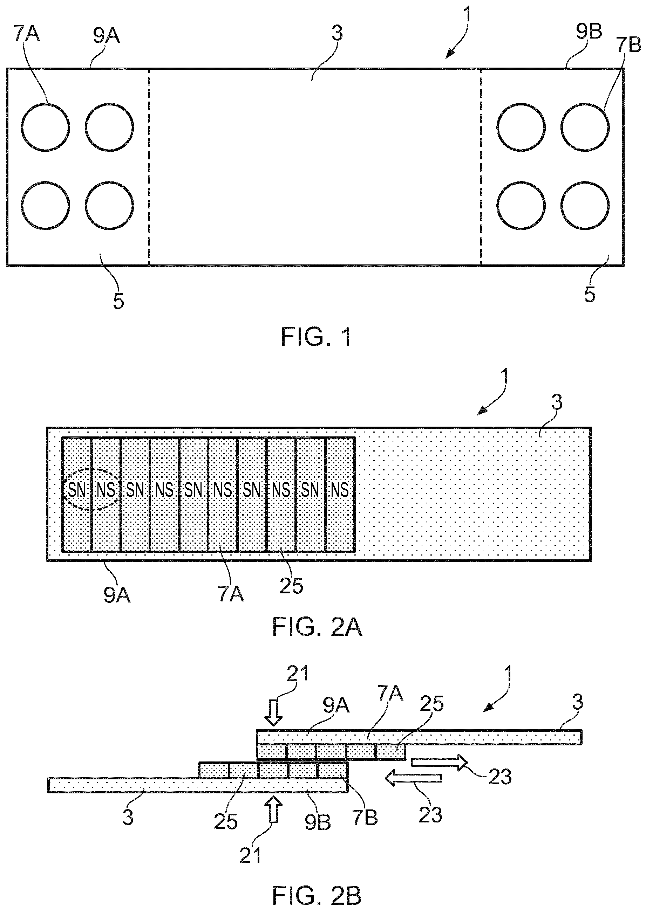

Fig. 1 schematically illustrates an apparatus 1 according to examples of the disclosure. The example apparatus 1 comprises a flexible member 3 and a magnetic arrangement 5. It is to be appreciated that the apparatus 1 may comprise additional components in other examples of the disclosure. For example the apparatus 1 may comprise one or more sensors for detecting one or more biometric parameters of the subject.

The flexible member 3 may be sized and shaped so as to enable it to be wrapped around part of a subject's body. The flexible member may be sized and shaped so that when it is wrapped around the part of the subject's body a first end portion 9A of the flexible member 3 overlaps a second end portion 9B of the flexible member 3. The flexible member 3 may be sized and shaped to fit around a subject's limb, around a subject's torso or around any other suitable part of the subject's body.

In the example of Fig. 1 the flexible member 3 is rectangular in shape so as to provide a strap. Other shapes of the flexible member 3 may be used in other examples of the disclosure. For example, the flexible member 3 could be arranged to provide a cuff or a sleeve. The shape of the flexible member 3 that is used may depend on the part of the subject's body that the apparatus 1 is intended to be attached to.

The flexible member 3 comprises a first end portion 9A and a second end portion 9B as indicated by the dashed lines. The first end portion 9A comprises a first edge of the flexible member 3 and also an area adjacent to the first edge. The second end portion 9B is provided at the opposite end of the flexible member 3 to the first end portion 9A. The second end portion 9B also comprises a second edge of the flexible member 3 and an area adjacent to the second edge.

In the example of Fig. 1 the first end portion 9A is the same size and shape as the second end portion 9B. In other examples the first end portion 9A and the second end portion 9B could have different sizes and/or shapes. The sizes and shapes of the end portions 9A, 9B may be dependent upon the part of the subject's body that the apparatus 1 is intended to be attached to, the size and shape of the flexible member 3, the type of magnetic arrangement 5 used or any other suitable factor.

The flexible member 3 may comprise any flexible material which is flexible enough to enable the flexible member 3 to be deformed around a subject so that the first end portion 9A overlaps at least some of the second end portion 9B. This may enable the flexible member 3 to be wrapped around the subject's body. The flexible member 3 may be flexible enough so that the flexible member 3 can be bent to fit tightly to the portion of the subject's body.

In some examples the flexible member 3 may comprise textile, polymer, leather or any other suitable material or combination of materials. In some examples the flexible member 3 could comprise a stretchable or elastic material or a chained structure made from a non-elastic material such as metal, or any other suitable flexible structure. In some examples the flexible member 3 may comprise a plurality of different materials. The different materials may be provided in different layers within the flexible member 3.

The magnetic arrangement 5 may comprise any means which provides a magnetic field that enables the first end portion 9A to be fastened to the second end portion 9B. When the first end portion 9A is fastened to the second end portion 9B the two end portions 9A, 9B are held tightly together so as to restrict relative movement of the two end portions 9A, 9B.

The magnetic arrangement 5 may comprise any suitable magnetic material. In some examples the magnetic arrangement 5 comprises magnetic ink. The magnetic ink may be deposited or printed onto the flexible member 3. The magnetic ink may comprise magnetic rare earth metals such as neodymium, alloys comprising rare earth metals or any other suitable magnetic material in forms of inks or powders. In some examples the magnetic ink may be magnetized after it has been deposited or printed onto the flexible member 3.

In some examples the magnetic arrangement 5 may be provided in a thin layer within the flexible member 3 so that the magnetic arrangement 5 is flexible enough to be deformed when the flexible member 3 is deformed. In other examples the magnetic arrangement 5 could be provided as a rigid portion in one or both of the end portions 9A, 9B of the flexible member 3.

The magnetic arrangement 5 may be embedded within the flexible member 3. The magnetic arrangement 5 may be embedded within the flexible member 3 so that the magnetic arrangement 5 is provided beneath the surface of the flexible member 3. This may prevent any magnets within the magnetic arrangement 5 from coming into direct contact with the subject or with other magnets. This may reduce the chance of the magnetic arrangement 5 from being damaged or worn out.

In the example apparatus 1 of Fig. 1 the magnetic arrangement 5 comprises at least a first magnet 7A provided in the first end portion 9A and at least a second magnet 7B provided in the second end portion 9B. In the example of Fig. 1 a plurality of first magnets 7A are provided in the first end portion 9A and a plurality of second magnets 7B are provided in the second end portion 9B. In the example of Fig. 1 four magnets 7A are provided in the first end portion 9A and four magnets 7B are provided in the second end portion 9B. Other numbers of magnets could be used in other examples of the disclosure.

In the example of Fig. 1 the pluralities of magnets 7A, 7B are arranged in two dimensional arrays comprising a plurality of columns and a plurality of rows. In the example of Fig, 1 both of the pluralities of magnets 7A, 7B comprise four magnets arranged in a two by two array. It is to be appreciated that other numbers of magnets and arrangements of magnets could be used in other examples of the disclosure.

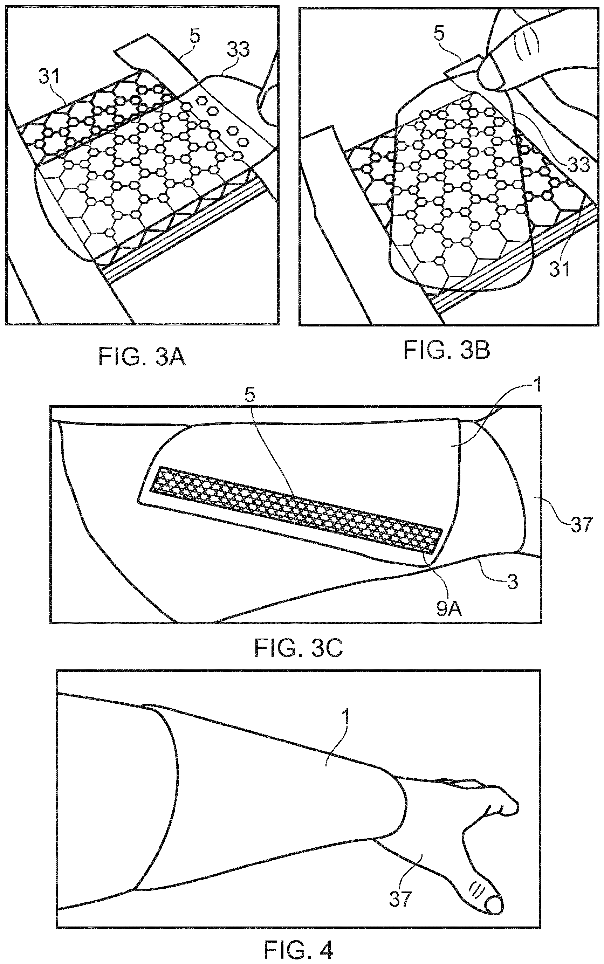

In some examples of the disclosure the magnetic arrangement 5 may comprise a pattern with at least two orders of rotational symmetry. The pattern could be formed by a plurality of magnets or by a single magnet. The pattern with at least two orders of rotational symmetry could be provided in the first end portion 9A and/or the second end portion 9B. In some examples the same pattern could be provided in both end portions 9A, 9B to enable step-tensioning functionality. In other examples different patterns could be used in the different end portions 9A, 9B. Where different patterns are used, the patterns may have some correspondence so that at least part of the first pattern can be aligned with parts of the second pattern when the two end portions 9A, 9B overlap. Examples of a magnetic arrangement 5 comprising a pattern with at least two orders of rotational symmetry are shown in more detail in Figs. 3A to 3C.

When the apparatus 1 is in use the flexible member 3 is wrapped around a portion of the subject's body so that the first end portion 9A overlaps at least part of of the second end portion 9B. The magnetic arrangement 5 is arranged so that the at least one magnet 7A in the first end portion 9A provides an attractive force for the at least one magnet 7B in the second end portion 9B. This attractive force retains the first end portion 9A in position relative to the second end portion 9B and secures the flexible member 3 in the wrapped configuration.

In examples of the disclosure the magnetic arrangement 5 comprises a plurality of magnets 7A, 7B within at least one end portion 9A, 9B of the flexible member 3. Where a plurality of magnets 7A, 7B are provided in at least one end portion 9A, 9B there may be a plurality of different configurations in which the magnetic forces between the respective magnets 7A, 7B act to secure the first end portion 9A in position relative to the second end portion 9B. This may enable the first end portion 9A and the second end portion 9B to be held together in a plurality of different positions so as to provide an adjustable apparatus 1 with step-tensioning functionality. The step-tensioning functionality enables the apparatus 1 to be arranged into a plurality of fastened positions.

Figs. 2A and 2B schematically illustrate sections of an apparatus 1 according to another example of the disclosure. Fig. 2A illustrates a plan view of a first end portion 9A of the flexible member 3 and Fig. 2B illustrates a cross section of the first end portion 9A overlapping the second end portion 9B.

In the example of Figs. 2A and 2B the magnetic arrangement 5 comprises a first plurality of magnets 7A provided in the first end portion 9A and a second plurality of magnets 7B provided in the second end portion 9B. The first plurality of magnets 7A and the second plurality of magnets 7B are provided in the same configuration in the example of Figs. 2A and 2B. In other examples the first plurality of magnets 7A and the second plurality of magnets 7B could be provided in different configurations.

In the example of Figs. 2A and 2B the plurality of magnets 7A and 7B comprises a plurality of magnetic blocks 25. The plurality of magnetic blocks may be individual magnets. A gap may be provided between adjacent magnetic blocks 25.

As shown in the plan view of Fig. 2A the magnetic blocks 25 are arranged in a linear array. The linear array comprises a single row of magnetic blocks. It is to be appreciated that in other examples the plurality of magnetic blocks 25 could be arranged in arrays having a plurality of rows and columns.

In the example of Figs. 2A and 2B the plurality of magnets 7A, 7B are arranged in a repelling configuration. In the repelling configuration the plurality of magnets 7A are positioned so that adjacent magnetic blocks 25 have repelling polarities. As shown in Fig. 2A each of the magnetic blocks 25 have a north pole and a south pole. The north and south poles are provided at the edges of the magnetic blocks 25. The magnetic blocks 25 are provided in alternating directions so that the north pole of a magnetic block 25 is adjacent to the north pole of a neighbouring magnetic block 25 and the south pole of the magnetic block 25 is adjacent to the south pole of a neighbouring magnetic block 25.

The repelling configuration provides for a magnetic field which has a large gradient in the region between two adjacent magnetic blocks 25. This may provide a large magnetic force on a magnet positioned in the region between two adjacent magnetic blocks 25.

Fig. 2B shows the flexible member 3 arranged so that the first end portion 9A overlaps the second end portion 9B. In this configuration the first plurality of magnets 7A and the second plurality of magnets 7B also overlap each other. The first plurality of magnets 7A and the second plurality of magnets 7B provide a magnetic field which acts to retain the first end portion 9A in position relative to the second end portion 9B.

If the second plurality of magnets 7B are positioned over the first plurality of magnets 7A so that a north pole in the second plurality of magnets 7B is aligned with a south pole in the first plurality of magnets 7A then a perpendicular magnetic force, as indicated by the arrows 21 acts to pull the two pluralities of magnets 7A, 7B towards each other. This fastens the respective end portions 9A, 9B of the flexible member 3 together.

The repelling configuration of the magnetic arrangement 5 also means that if the first plurality of magnets 7A and the second plurality of magnets 7B are not aligned with each other a horizontal magnetic force, as indicated by the arrows 23, acts to push the pluralities of magnets 7A, 7B into a position in which the magnets are aligned. For example if a north pole in the second plurality of magnets 7B is positioned overlaying or close to a north pole in the first plurality of magnets 7A the strong magnetic gradient between the adjacent magnetic blocks 25 provides a force which pushes the pluralities of magnets 7A, 7B towards an aligned configuration. This helps the apparatus 1 to be arranged into a fastening position.

The example magnetic arrangement 5 of Figs. 2A and 2B enables a discrete number of fastening positions to be provided. This enables the flexible member 3 to be adjusted into different configurations corresponding to the different fastening positions. The different fastening positions can be selected based on the part of the subject's body the apparatus 1 has been attached to, how tight the subject wants the apparatus 1 to be or any other suitable factors.

Figs. 3A to 3C illustrate another example apparatus 1 according to another example of the disclosure. In the example apparatus 1 of Figs. 3A to 3C the magnetic arrangement 5 comprises a two dimensional array. Fig. 3A illustrates a magnetic arrangement 5 in a first orientation, Fig. 3B illustrates a magnetic arrangement 5 in a second orientation and Fig. 3C illustrates the apparatus 1 in use wrapped around an arm 37 of a subject. The flexible member 3 is not shown in Figs. 3A and 3B.

As shown in Figs. 3A and 3B the magnetic arrangement 5 comprises a pattern with at least two orders of rotational symmetry. In the example of Figs. 3A to 3C a first pattern 31 is provided in the first end portion 9A of the flexible member 3 and a second pattern 33 is provided in the second end portion 9B of the flexible member 3. The first pattern 31 is different to the second pattern 33. In other examples the first pattern 31 and the second pattern 33 could be the same.

The first pattern 31 comprises a tessellated array comprising a plurality of polygons. In the example of Figs. 3A to 3C the polygons comprise hexagons. The array of hexagons has six orders of rotational symmetry. Other shapes and arrangements could be used for the first pattern 31 in other examples of the disclosure.

The first pattern 31 comprises a tessellated pattern so that the pattern can cover the first end portion 9A of the flexible member 3. In other examples a non-tessellated pattern which may comprise one or more gaps or discontinuities may be used.

The second pattern 33 is shown on a transparent substrate in Figs. 3A and 3B so as to show the alignment between the first pattern 31 and the second pattern 33. It is to be appreciated that both the first pattern 31 and the second pattern 33 could be provided within the same flexible member 3 in examples of the disclosure.

In the example of Figs. 3A to 3C the second pattern 33 comprises a plurality of individual magnets arranged in array so that the individual magnets in the second pattern 33 align with vertices of the polygons in the first pattern 31. In the example of Figs. 3A to 3C the second pattern 33 comprises a plurality of individual magnets arranged in a hexagonal array which are spaced to align with the vertices of the hexagons in the first pattern 31.

Fig. 3A shows the second pattern 33 being positioned overlaying the first pattern 31. This shows that the magnets in the second pattern 33 can be positioned to align with the vertices of the hexagons in the first pattern 31. Fig. 3B shows the second pattern 33 having been rotated through 60 degrees relative to the first pattern 31. Even though the patterns 31, 33 have been rotated relative to each other the magnets in the second pattern 33 are still aligned with the vertices of the hexagons in the first pattern 31 due to the rotational symmetry of the patterns 31, 33.

The patterns 31, 33 of the magnetic arrangement 5 could be formed from any suitable materials. In some examples the patterns could be printed or otherwise deposited onto the flexible member 3. In such examples the ink could be magnetized after the patterns 31, 33 have been printed.

In the examples of Fig. 3A to 3C the magnetic poles could be provided perpendicular to the surface of the flexible member 3. That is, the magnetic poles could be provided perpendicular to the areas covered by the respective patterns. The magnetic poles could be arranged so that when the apparatus 1 is arranged with the first end portion 9A overlapping the second end portion 9B an attractive magnetic force is provided between the two magnetic patterns 31, 33 so as to secure the two end portions 9A, 9B relative to each other.

Fig. 3C shows an apparatus 1 comprising a magnetic arrangement 5 as shown in Figs 3A and 3B. The magnetic patterns 31, 33 of the magnetic arrangement 5 may be embedded within the flexible member 3 of the apparatus 1.

The apparatus 1 provides a sleeve or cuff which is wrapped around the lower arm 37 of the subject. The sleeves or cuff may be used to measure biometric parameters of the subject. For example, it may comprise one or more sensors arranged to measure parameters such as heart rate or blood pressure.

As the magnetic arrangement 5 has a plurality of orders of rotational symmetry this enables the first end portion 9A of the flexible member 3 to be positioned at an angle relative to the second end portion 9B. This may enable the apparatus 1 to be fastened in a well-fitting arrangement. In the well-fitting arrangement the sleeve or cuff may be in close contact with the subject's skin along the length of the lower arm 37. This may make the apparatus 1 more comfortable for the subject to wear and may also improve the accuracy of any measurements made by sensors within the apparatus 1.

Fig. 4 illustrates another example apparatus 1 in use. The example apparatus 1 of Fig. 4 could have magnetic arrangements as shown in and of Figs. 1 to 3C, or any other suitable magnetic arrangement.

In the example of Fig. 4 the apparatus 1 is wrapped around the lower arm 37 of the subject. The apparatus 1 is arranged so that it has a different circumference at different points along the length of the apparatus 1. That is the apparatus 1 is arranged to have a smaller circumference around the wrist of the subject and to have a larger circumference at an end closer to the elbow of the subject. The magnetic arrangement 5 may be arranged so that the respective end portions of the flexible member 3 are fastened together along the length of subject's arm.

In examples of the disclosure use of the magnetic arrangement 5 within the flexible member 3 enables the end portions 9A, 9B of the flexible member 3 to be fastened to each other so that the first end portion 9A can be held firmly in position relative to the second end portion 9B. The magnetic arrangement 5 may have a plurality of configurations in which the magnetic field of the magnetic arrangement 5 acts so that the first end portion 9A will be fastened to the second end portion 9B. This provides for an adjustable apparatus 1.

In some examples of the disclosure the magnetic arrangement 5 may be printed or otherwise deposited into the flexible member 3. This may provide for a large degree in freedom in the design of the magnetic arrangement 5. The may enable complex geometries to be used for the magnetic arrangement 5 which may provide for a more secure fastening, or a fastening that is easier to adjust.

In some examples the apparatus 1 could be used for measuring biometric parameters of the subjects. For example the apparatus 1 could comprise sensors which enable parameters such heart rate and blood pressure to be monitored. In such examples the magnetic arrangement may enable the apparatus 1 to be held securely relative to the subject's body. This may prevent or restrict movement of the apparatus 1 while the biometric parameters are being measured which may provide for more accurate and/or reliable measurements.

The term "comprise" is used in this document with an inclusive not an exclusive meaning. That is any reference to X comprising Y indicates that X may comprise only one Y or may comprise more than one Y. If it is intended to use "comprise" with an exclusive meaning then it will be made clear in the context by referring to "comprising only one..." or by using "consisting".

In this brief description, reference has been made to various examples. The description of features or functions in relation to an example indicates that those features or functions are present in that example. The use of the term "example" or "for example" or "may" in the text denotes, whether explicitly stated or not, that such features or functions are present in at least the described example, whether described as an example or not, and that they can be, but are not necessarily, present in some of or all other examples. Thus "example", "for example" or "may" refers to a particular instance in a class of examples. A property of the instance can be a property of only that instance or a property of the class or a property of a subclass of the class that includes some but not all of the instances in the class. It is therefore implicitly disclosed that a feature described with reference to one example but not with reference to another example, can where possible be used in that other example but does not necessarily have to be used in that other example.

Although embodiments of the present invention have been described in the preceding paragraphs with reference to various examples, it should be appreciated that modifications to the examples given can be made without departing from the scope of the invention as claimed. For instance, in the examples described above the magnetic arrangement 5 comprises one or more magnets 7A provided in the first end portion 9A and also one or more magnets 7B provided in the second end portion 9B. In other examples the first end portion 9A could comprise one or more magnets 7A while the second end portion 9B could comprise a material, such as ferromagnetic material, which is attracted to the magnets 7A but is not itself magnetized.

Features described in the preceding description may be used in combinations other than the combinations explicitly described.

Although functions have been described with reference to certain features, those functions may be performable by other features whether described or not.

Although features have been described with reference to certain embodiments, those features may also be present in other embodiments whether described or not.

Whilst endeavoring in the foregoing specification to draw attention to those features of the invention believed to be of particular importance it should be understood that the Applicant claims protection in respect of any patentable feature or combination of features hereinbefore referred to and/or shown in the drawings whether or not particular emphasis has been placed thereon.

## Claims

1. An apparatus comprising:
a flexible member arranged to be wrapped around part of a subject's body, the flexible member comprising a first end portion and a second end portion;
a magnetic arrangement embedded within the flexible member wherein the magnetic arrangement comprises at least one magnet positioned within the first end portion of the flexible member and at least one magnetic portion positioned within the second end portion of the flexible member; and
wherein the magnetic arrangement is arranged to hold the first end portion of the flexible member in position relative to the second end portion when the flexible member is bent so that the first end portion and the second end portion overlap.

2. An apparatus as claimed in claim 1 wherein the magnetic arrangement comprises a plurality of magnets arranged in an array comprising a plurality of columns and a plurality of rows.

3. An apparatus as claimed in any preceding claim wherein the magnetic arrangement comprises a pattern with at least two orders of rotational symmetry.

4. An apparatus as claimed in any preceding claim wherein the magnetic arrangement comprises a tessellated array comprising a plurality of polygons positioned within the first end portion of the flexible member and a plurality of magnets arranged to align with vertices of the polygons positioned within the second end portion of the flexible member.

5. An apparatus as claimed in any preceding claim wherein the magnetic arrangement comprises a hexagonal array comprising a plurality of polygons positioned within the first end portion of the flexible member and a plurality of magnets arranged to align with vertices of the hexagons positioned within the second end portion of the flexible member.

6. An apparatus as claimed in any preceding claim wherein a plurality of magnets are provided within least one portion of the flexible member and the plurality of magnets are arranged so that adjacent magnets within have repelling polarities.

7. An apparatus as claimed in any of claims 1 to 5 wherein the at least one magnet positioned at the first end portion has a first polarity and at least one magnet positioned at the second end portion has a different polarity.

8. An apparatus as claimed in any preceding claim wherein the magnetic arrangement is printed onto the flexible member.

9. An apparatus as claimed in any preceding claim wherein the at least one magnetic portion positioned within the second end portion of the flexible member comprises one or more magnets.

10. An apparatus as claimed in any preceding claim wherein the flexible member is arranged to be wrapped around a portion of a subject's body.

11. An apparatus as claimed in any preceding claim wherein the flexible member comprises textile.

12. An apparatus as claimed in any preceding claim wherein the flexible member comprises polymer.

13. An apparatus as claimed in any preceding claim wherein the apparatus comprises one or more sensors arranged to detect a biometric parameter of the subject.

14. A wearable electronic device comprising an apparatus as claimed in any preceding claim.
